# EUROPEAN PATENT SPECIFICATION

(11) **EP 0 647 232 B1**
(45) Date of publication and mention of the grant of the patent: **28.04.1999**
(21) Application number: 93913877.2
(22) Date of filing: 13.05.1993
(51) Int. Cl.: C12N 9/99, C12Q 1/68

(54) **DEPROTEINIZATION WITH AZLACTONE-FUNCTIONAL SUPPORTS**
ENTPROTEINISIERUNG MIT AZLACTON-GEKOPPELTEN FUNKTIONSTRÄGERN
DEPROTEINISATION A L'AIDE DE SUPPORTS FONCTIONNELS D'AZLACTONE

(30) Priority: 23.06.1992 US 902978
(43) Date of publication of application: 12.04.1995
(73) Proprietor: MINNESOTA MINING AND MANUFACTURING COMPANY, St. Paul, Minnesota 55133-3427 (US)
(72) Inventor: BITNER, Rex, M., Saint Paul, MN 55133-3427 (US); CHAN-WHA, Kim, Saint Paul, MN 55133-3427 (US); WILLIAMS, Michael, G., Saint Paul, MN 55133-3427 (US)
(74) Representative: Warcoin, Jacques
(86) International application number: US9304576
(87) International publication number: WO9400464

(56) References cited:
- EP-A- 0 392 735
- EP-A- 0 392 783
- WO-A-90/10637
- WO-A-92/07879

## Description

### FIELD OF THE INVENTION

This invention relates to a method for controlling restriction enzyme activities in biological fluid comprising non-proteinaceous, genetic material and restriction enzyme.

### BACKGROUND OF THE INVENTION

Deproteinization is often an essential requirement in the production or separation of high quality non-proteinaceous materials. Non-proteinaceous materials include polysaccharides, steroids, alkaloids, lipids, and genetic materials such as nucleic acids, nucleotides, plasmids. However, a major difficulty occurs because of a lack of a sufficient driving force to differentiate non-proteinaceous materials from proteinaceous materials. Proteinaceous materials include amino acids, peptides, proteins, glycoproteins, lipoproteins, and others.

Current deproteinization processes, such as solvent precipitation, acid precipitation, salting-out procedures, ionexchange adsorption, heat denaturation, ultra-filtration, ultra-centrifugation, and dialysis, are time consuming and costly or ineffective.

U.S. Patent 4,421,653 discloses a process for the deproteinization of biological fluids by removing the water insoluble phase after adding to the fluid a water insoluble crosslinked polycarbonic polymer. A water insoluble cross-linked diamine (ethylenediamine or hexamethylenediamine) polycarbonic acid is one of the deproteinizing agents employed.

However, the polyvalent cationic surface of the diamine polycarbonic acids provides non-specific adsorption of non-proteinaceous materials, such as nucleic acids and polysaccharides, which should be retained in the solution after deproteinization process for further recovery or analysis.

Additionally, U.S. Patent 4,923,978 discloses a process for separating proteinaceous materials from nucleic acids involving contacting a solution containing the proteinaceous materials and nucleic acids with solid phase extraction materials capable of binding proteins to form bound and unbound fractions and then isolating the unbound fraction containing the nucleic acids. Rehydrated silica gel is one of the solid phase extraction materials employed, but the surface of the silica gel must be freed of polyvalent cationic species if nucleic acids are to be prevented from being adsorbed on the surface of the silica particles. Three methods are disclosed to achieve a solid phase extraction material which is useful. But such methods involve difficult and time consuming processes.

U.S. Patent 5,004,806 (Kung) discloses a method to remove proteins from a solution containing polynucleotides and proteins by filtering the solution through a nitrocellulose membrane at neutral or basic pH. However, this method is confined to filtration techniques which do not completely deproteinize the solution nor provide efficient DNA retention in the filtrate when using neutral pH values.

European Patent Publication 0 392 735(A2) discloses azlactone-functional polymeric supports useful for the attachment of functional materials to provide novel adduct supports. The adduct supports are useful as complexing agents, catalysts, polymeric reagents, chromatographic supports, and as enzyme- or other biologically active supports.

European Patent Publication 0 392 783(A2) discloses azlactone graft copolymers which can immobilize proteins for later use.

### SUMMARY OF THE INVENTION

The problem of finding a method for controlling restriction enzyme activities in biological fluid comprising non-proteinaceous, genetic material and restriction enzyme in an effective and efficient manner is solved by the use of azlactone-functional supports which selectively interact with azlactone-reactive amine moieties in proteinaceous materials. Amine moieties are a differentiating factor between proteinaceous materials and non-proteinaceous materials.

Azlactone-functional supports selectively separate proteinaceous materials from non-proteinaceous materials. This separation can be made in fluids where both non-proteinaceous materials and proteinaceous materials are found, especially in biological fluids. Nonlimiting examples of biological fluids include whole blood, plasma, sera, lymph, bile, urine, fermentation liquor, spinal fluid, sputum, sweat, microbial culture filtrate, cell lysate, biological extracts, and fluid preparation from biological tissue. Preferably, for the production of high quality non-proteinaceous materials or for the preparation of analytical or diagnostic test samples, deproteinization is achieved by the binding of proteinaceous material to azlactone-functional supports.

A method of the invention comprises controlling restriction enzyme activities in a biological fluid comprising non-proteinaceous, genetic material and restriction enzyme. The method comprises (a) contacting an azlactone-functional support with biological fluid; (b) inactivating the restriction enzyme in the biological fluid by binding the restriction enzyme to the azlactone-functional support; and (c) separating the azlactone-functional support from the biological fluid to remove inactivated restriction enzyme from the biological fluid.

It is a feature of the present invention that non-proteinaceous genetic materials are separated from enzyme restriction materials by binding of the enzyme restriction materials to azlactone-functional supports.

It is another feature of the present invention that deproteinization of biological fluids is useful in the production or separation of non-proteinaceous genetic materials.

It is another feature of the present invention that deproteinization of biological fluids is useful for the preparation of analytical or diagnostic test samples.

It is another feature of the present invention that azlactone-functional supports can separate positively charged proteinaceous material, negatively charged proteinaceous material, and uncharged proteinaceous material from non-proteinaceous, genetic material.

It is an advantage of the present invention that the separation of proteinaceous material from non-proteinaceous, genetic material according to the present invention is achieved in an effective and efficient manner.

It is another advantage of the present invention that biologically active non-proteinaceous, genetic material separated according to the process of the present invention retains its biological activity and is otherwise unaffected by methods of the present invention.

Embodiments of the invention follow a brief description of the drawing.

### BRIEF DESCRIPTION OF THE DRAWING

Fig. 1 is a graph showing the adsorption and removal of a proteinaceous material from a solution using azlactone-functional particles.

Fig. 2 is a graph showing adsorption kinetics of two proteinaceous materials on azlactone-functional particles.

### EMBODIMENTS OF THE INVENTION

### Azlactone-Functional Supports

Azlactone-functional supports are materials having units of the formula: 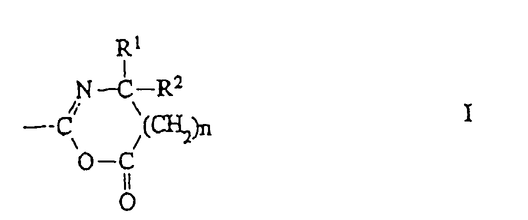 wherein R¹ and R² independently can be an alkyl group having 1 to 14 carbon atoms, a cycloalkyl group having 3 to 14 carbon atoms, an aryl group having 5 to 12 ring atoms, an arenyl group having 6 to 26 carbon and 0 to 3 S, N, and nonperoxidic O heteroatoms, or R¹ and R² taken together with the carbon to which they are joined can form a carbocyclic ring containing 4 to 12 ring atoms, and n is an integer 0 or 1.

Azlactone-functional supports useful in the methods of the present invention can be in the form of an article comprising azlactone-functional polymer or an azlactone-functional polymer coated on at least one surface of a substrate. Nonlimiting examples of azlactone-functional articles include films, webs, membranes, particles (such as beads), articles (such an microtiter wells or test tubes), and other structures from which the azlactone-functional unit of formula I above can be available for interaction with proteinaceous materials.

The structure of various polymeric supports having azlactone functional units of formula I above and their methods of preparation are disclosed in U.S. Patent 4,871,824 (Heilmann, et al.) and European Patent Publication 0 392 735 (Heilmann, et al.). Such azlactone-functional materials can be used in the method of the present invention.

Azlactone graft copolymers and methods of their preparation are disclosed in U.S. Patent 5,013,795 (Coleman, et al.) and European Patent Publication 0 392 783. Such azlactone-functional materials are useful in the method of the present invention.

Azlactone-functional supports are also disclosed in International Application serial No. US 92/07659 (Rasmussen et al.) and in International Application Serial No. US 93/04555 (Gagnon et al.) (Attorney Docket 45471PCT2A).

When the azlactone-functional support is in the form of particles, the size of the particles can be from about 0.1 to 1,000 µm and preferably 0.5 to 100 µm. The particles can be porous or non-porous. When porous, the porosity of the particles can range from about 1 to about 1,000 Angstroms and preferably from about 10 to about 500 Angstroms.

When in the form of a membrane or web, the porosity of the azlactone-functional supports should be of a size to permit access of proteinaceous materials to surfaces of the membrane or web, and generally can have physical characteristics described in Rasmussen et al. and Gagnon et al. identified above.

The degree of deproteinization according to the present invention depends on the density of azlactone units of formula I above on the surface of the azlactone-functional support, the available surface area of the support having azlactone-functionality, and when the support is in the form of a particle, the size of the particle.

### DEPROTEINIZATION HETHODS

Azlactone-functional supports can be used to separate restriction enzyme materials from non-proteinaceous materials in several manners.

When azlactone-functional supports are in the form of particles, they can be added to a vessel containing proteinaceous and non-proteinaceous materials in a fluid. After gentle agitation between about 1 to 10 minutes, such particles can be removed by centrifugation, skimming, filtration or sedimentation. Removal of azlactone-containing particles from the vessel leaves a deproteinized supernatant containing non-proteinaceous materials for further processing.

Azlactone-functional supports in the form of particles can also be packed into columns through which fluids, especially biological fluids, can be drawn using a variety of chromatographic techniques. Non-limiting examples of such chromatographic techniques include high pressure liquid chromatography, spin column chromatography, mini column chromatography, and the like.

Azlactone-functional supports in the form of membranes or webs can be used in the method of the present invention in microtiter wells, filters, dialysis tubing, linings on walls of vessels containing such proteinaceous materials or transport structures having such proteinaceous materials passing through, and the like.

When the azlactone-functional supports are in the form of articles such as microtiter wells or test tubes, proteinaceous material is retained in the article and the deproteinized supernatant is removed for further processing.

When it is desired to remove proteinaceous material from the biological fluids described above, the degree of deproteinization can vary according to the number of azlactone-functional groups that are available and the amount of proteinaceous material in the biological fluid. The temperature, pH-value, ionic strength, and buffering condition are, in principle, not critical. However; the temperature generally lies between 0 to 100°C, preferably between 4°C and 40°C, and the pH-value between 0 and 11, desirably between 4 and 11, and preferably between 6 and 10. When deproteinizing mixtures containing DNA, it is perferable to keep the pH around 7 to 8.5 to retain biological activity. When deproteinizing mixtures containing RNA, it is preferable to keep the pH around 5.5 to 7 to retain biological activity. When deproteinizing mixtures containing high-molecular weight polymers such as nucleic acids or polysaccharides, it is preferable that the agitation be gentle and avoid processing which could generate shear forces.

Further description of the details of the invention is found in the following examples.

### EXAMPLE 1

### Deproteinization of Biological Fluids Containing Recombinant DNA

When performing recombinant DNA manipulations of biological extracts, it is often necessary to alter DNA molecules using one enzyme under a particular set of conditions (such as pH value and MgCl₂, NaCl, KCl concentrations), and then to additionally modify the DNA using a second enzyme requiring a different set of conditions. One of the chief difficulties in performing these series of reactions, is that the first enzyme possesses altered specificity under the second set of conditions.

A common example is that of the restriction enzyme EcoRI, which cuts the DNA sequence GAATTC when-used in standard conditions such as 50 mH Tris (pH 8.0), 10 mM HgCl₂, and 100 mM NaCl. However, under lower salt concentration or higher pH, EcoRI will alter its specificity and cut the sequence AATT. This is commonly referred to as EcoRI* (EcoRI star) activity, and results in undesired degradation of the DNA molecules. Hence, the desirability of destruction/removal of EcoRI activity before changing the reaction conditions for the addition of a second enzyme.

The most common method of removing enzyme activity is through treatment of the solution with phenol. Unfortunately, the phenol must then be removed through extraction with chloroform, and the DNA precipitated with ethanol (see Molecular Cloning, T. Haniatis, E. F. Fritsch and J. Sambrook, Cold Spring Harbor, 1982). The procedure is laborious, and produces hazardous chemical wastes with expensive disposal costs.

The deproteinization of the DNA/enzyme mixture using azlactone-functional supports is an attractive alternative to phenol treatment. A preferred method is the use of azlactone-functional particles (prepared according to the method of Process III and Examples 5A-5K of European Patent Publication 0 392 735), either by direct addition of the beads to the DNA/enzyme mixture and their removal by centrifugation, or by passing the DNA/enzyme mixture through a minicolumn containing azlactone network beads.

As an example of this procedure, 1.0 µg of phage lambda DNA was cut with restriction enzyme HindIII in 50 mM Tris-HCl (pH 8.0), 10 mM HgCl₂ and 50 mM NaCl in a volume of 30 µl by incubation at 37°C for 40 minutes. All reactions described were in 400 µl polypropylene tubes, distributed by Biorad, (Richmond, CA), catalog number 223-9503. The reaction mixture was then passed through a minicolumn (a sterile, plastic automatic pipeter tip containing 1 mg of azlactone-functional beads). The solution was kept in contact with the azlactone-functional beads for 10 seconds, and the solution was slowly passed through the column during an additional 5 seconds. 0.2 µg of plasmid pBR322 DNA was added to 15 µl of the phage lambda/HindIII solution and incubated for an additional 4 hours at 37°C, then analyzed by gel electrophoresis. The pBR322 DNA was not digested by any remaining HindIII activity in the reaction mix, indicating that the HindIII was completely removed/inactivated by the azlactone treatment.

The NaCl concentration of the remaining 15 µl of HindIII digested lambda DNA was raised from 50 mM to 100 mM, and the DNA was digested with EcoRI for 40 minutes at 37°C, and analysis by gel electrophoresis indicated complete digestion. This shows that the exposure of the DNA sample to the azlactone-functional beads had no deleterious effect on subsequent EcoRI digestion.

A sample of the HindIII EcoRI digested DNA was run through an azlactone-functional bead minicolumn (as previously described), and the DNA was ethanol precipitated, and ligated overnight with T4 DNA ligase. Gel electrophoresis showed complete ligation of the sample, indicating that the single stranded ends of the DNA remained intact after exposure to the azlactone-functional beads.

This example shows that azlactone-functional beads can be used to remove undesired enzyme activities from biological samples. It has worked equally well with less purified DNA, prepared from a "10 minute" DNA purification system, described in Biotechniques (Feb., 90): volume 8, p172-173. The DNA is apparently unaffected by its exposure to azlactone-functional beads prepared according to Process III described above, and can be digested with additional enzymes, or ligated efficiently by T4 DNA ligase.

### EXAMPLE 2

### Deproteinization of Biological Fluids Containing Plasmids

As additional evidence that DNA is unaffected by exposure to azlactone-functional support, plasmid pUC118 was cut with Pst I as previously described. The sample was exposed to azlactone-functional beads as described, and the 100% cut pUC118 was ethanol precipitated, and ligated with T4 DNA ligase. the Pst I site cuts pUC118 in the sequence coding for beta-galactosidase, and any alteration of the nucleotide bases in this sequence would result in a bacterial transformant which is Lac minus. The frequency of Lac minus transformants after azlactone exposure was between 0.2 to 0.5%, similar to the 0.2 to 0.9% seen in the phenol treated controls. Similar results were obtained with HindIII digested pUC118. This further demonstrates that DNA sequences are unaltered by exposure to azlactone network beads.

## Claims

1. A method for controlling restriction enzyme activities in biological fluid comprising non-proteinaceous, genetic material and restriction enzyme, comprising :
a) contacting azlactone-functionnal support with said biological fluid;
b) inactivating said restriction enzyme in said biological fluid by binding said restriction enzyme to said azlactone-functional support; and
c) separating said azlactone-functional support from said biological fluid to remove inactivated restriction enzyme from said biological fluid whereby said non-proteinaceous, genetic material retain biological activity.

2. The method according to claim 1, wherein the method further comprises the steps of :
d) digesting the non-proteinaceous, genetic material with a second enzyme; and
e) ligating the digested, non-proteinaceous, genetic material with a ligase.

3. The method according to claims 1 or 2, wherein said non-proteinaceous, genetic material comprises nucleic acids.

4. The method according to any of claims 1 to 3 wherein said azlactone-functional support is a material having units of the formula : 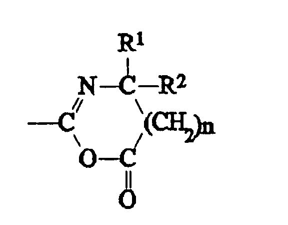 wherein R¹ and R² independently can be an alkyl group having 1 to 14 carbon atoms, a cycloalkyl group having 3 to 14 carbon atoms, an aryl group having 5 to 12 ring atoms, an arenyl group having 6 to 26 carbon atoms and 0 to 3 S,N, and nonperoxidic 0 heteroatoms, or R1 or R2 taken together with the carbon to which they are joined can form a carbocyclic ring containing 4 to 12 ring atoms, and n is an integer 0 or 1.

5. The method according to any of claims 1 to 4, wherein said azlactone-functional support are particles mixed into the biological fluid for binding of restriction enzyme materials to said particles, and wherein said particles are removed from said biological fluid after said binding.

6. The method according to any of claims 1 to 4, wherein said azlactone-functional support is an article which contacts said biological fluid for binding of restriction enzyme materials to said article, and wherein said azlactone-functional article comprises a membrane or web.

7. The method according to any claims 1 to 5 wherein said biological fluid comprises whole blood, plasma, sera, lymph, bile, urine, fermentation liquor, spinal fluid, sputum, sweat, microbial culture filtrate, biological extracts, cell lysate and combinations thereof.

8. The method according to any of claims 1 to 7, wherein said restriction enzyme material binds to said azlactone-functional support at a pH value of between 0 and 11 and at a temperature from 0°C to 100°C within a reaction time of 1 to 10 minutes.

9. The method according to any of claims 1 to 8 wherein said separating comprises centrifugation, skimming, filtration, or sedimentation.

## Patentansprüche

1. Verfahren zur Steuerung der Aktivitäten von Restriktionsenzymen in einer biologischen Flüssigkeit, die nichtproteinartiges genetisches Material und Restriktionsenzym umfaßt, umfassend:
a) In-Kontakt-Bringen von Azlacton-funktionellem Träger mit der biologischen Flüssigkeit;
b) Inaktivieren des Restriktionsenzyms in der biologischen Flüssigkeit durch Binden des Restriktionsenzyms an den Azlacton-funktionellen Träger; und
c) Abtrennen des Azlacton-funktionellen Trägers aus der biologischen Flüssigkeit, so daß inaktiviertes Restriktionsenzym aus der biologischen Flüssigkeit entfernt wird, wobei das nichtproteinartige genetische Material seine biologische Aktivität beibehält.

2. Verfahren gemäß Anspruch 1, wobei das Verfahren weiterhin die folgenden Schritte umfaßt:
d) Abbauen des nichtproteinartigen genetischen Materials mit einem zweiten Enzym; und
e) Ligasieren des abgebauten nichtproteinartigen genetischen Materials mit einer Ligase.

3. Verfahren gemäß Anspruch 1 oder 2, wobei das nichtproteinartige genetische Material Nucleinsäuren umfaßt.

4. Verfahren gemäß einem der Ansprüche 1 bis 3, wobei der Azlacton-funktionelle Träger ein Material ist, das Einheiten der Formel 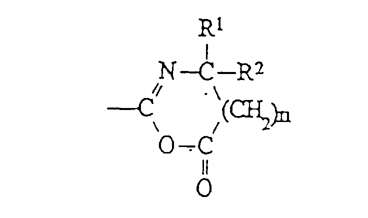 aufweist, wobei R¹ und R² unabhängig eine Alkylgruppe mit 1 bis 14 Kohlenstoffatomen, eine Cycloalkylgruppe mit 3 bis 14 Kohlenstoffatomen, eine Arylgruppe mit 5 bis 12 Ringatomen, eine Arenylgruppe mit 6 bis 26 Kohlenstoffatomen sowie 0 bis 3 S-, N- und nichtperoxidischen O-Heteroatomen sein können oder R¹ und R² zusammen mit dem Kohlenstoffatom, an das sie gebunden sind, einen carbocyclischen Ring bilden können, der 4 bis 12 Ringatome enthält, und n eine der ganzen Zahlen 0 oder 1 ist.

5. Verfahren gemäß einem der Ansprüche 1 bis 4, wobei es sich bei dem Azlacton-funktionellen Träger um Teilchen handelt, die in die biologische Flüssigkeit eingemischt werden, um Restriktionsenzymmaterialien an die Teilchen zu binden, wobei die Teilchen nach der Bindung aus der biologischen Flüssigkeit entfernt werden.

6. Verfahren gemäß einem der Ansprüche 1 bis 4, wobei es sich bei dem Azlacton-funktionellen Träger um einen Artikel handelt, der mit der biologischen Flüssigkeit in Kontakt steht, um Restriktionsenzymmaterialien an den Artikel zu binden, wobei der Azlacton-funktionelle Artikel eine Membran oder ein Vlies umfaßt.

7. Verfahren gemäß einem der Ansprüche 1 bis 5, wobei die biologische Flüssigkeit Vollblut,Plasma, Serum, Lymphe, Galle, Urin, Fermentationsflüssigkeit, Liquor, Sputum, Schweiß, Mikrobenkulturfiltrat, biologische Extrakte, Zellysat und Kombinationen davon umfaßt.

8. Verfahren gemäß einem der Ansprüche 1 bis 7, .wobei das Restriktionsenzymmaterial bei einem pH-Wert zwischen 0 und 11 und bei einer Temperatur von 0°C bis 100°C innerhalb einer Reaktionszeit von 1 bis 10 Minuten an den Azlacton-funktionellen Träger bindet.

9. Verfahren gemäß einem der Ansprüche 1 bis 8, wobei das Abtrennen eine Zentrifugation, ein Abschöpfen, eine Filtration oder Sedimentation umfaßt.

## Revendications

1. Procédé pour réguler l'activité d'enzymes de restriction dans un fluide biologique comprenant un matériau génétique non-protéiné et une enzyme de restriction, qui comprend :
a) la mise en contact d'un support à fonctionnalité azlactone avec ledit fluide biologique ;
b) l'inactivation de ladite enzyme de restriction dans ledit fluide biologique par fixation de ladite enzyme de restriction audit support à fonctionnalité azlactone ; et
c) la séparation dudit support à fonctionnalité azlactone d'avec ledit fluide biologique pour éliminer l'enzyme de restriction inactivée dudit fluide biologique, de façon que ledit matériau génétique non-protéine conserve son activité biologique.

2. Procédé selon la revendication 1, dans lequel le procédé comprend en outre les étapes :
d) de digestion du matériau génétique non-protéiné avec une deuxième enzyme ; et
e) de ligature, avec une ligase, du matériau génétique non-protéine digéré.

3. Procédé selon les revendications 1 ou 2, dans lequel ledit matériau génétique non-protéiné comprend des acides nucléiques.

4. Procédé selon l'une quelconque des revendications 1 à 3, dans lequel ledit support à fonctionnalité azlactone est un matériau ayant des unités de formule : 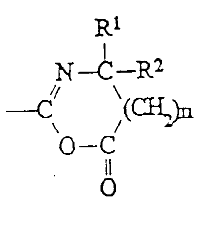 dans laquelle R¹ et R² représentent chacun indépendamment de l'autre un groupe alkyle ayant de 1 à 14 atomes de carbone, un groupe cycloalkyle ayant de 3 à 14 atomes de carbone, un groupe aryle ayant de 5 à 12 atomes nucléaires, un groupe arényle ayant de 6 à 26 atomes de carbone et de 0 à 3 hétéroatomes S, N ou O non-peroxydes, ou encore R¹ et R², avec l'atome de carbone auquel ils sont liés, peuvent former un noyau carbocyclique contenant de 4 à 12 atomes nucléaires, et n est un entier valant 0 ou 1.

5. Procédé selon l'une quelconque des revendications 1 à 4, dans lequel ledit support à fonctionnalité azlactone est constitué de particules mélangées à un fluide biologique pour fixation desdits matériaux d'enzymes de restriction auxdites particules, et dans lequel lesdites particules sont éliminées dudit fluide biologique après ladite fixation.

6. Procédé selon l'une quelconque des revendications 1 à 4, dans lequel ledit support à fonctionnalité azlactone est un article qui entre en contact avec ledit fluide biologique pour fixation des matériaux d'enzyme de restriction audit article, et dans lequel ledit article à fonctionnalité azlactone comprend une membrane ou une bande.

7. Procédé selon l'une quelconque des revendications 1 à 5, dans lequel ledit fluide biologique comprend le sang total, le plasma, les sérums, la lymphe, la bile, l'urine, le liquide de fermentation, le fluide spinal, les crachats, la sueur, les filtrats de cultures microbiennes, les extraits biologiques, les lysats cellulaires et leurs combinaisons.

8. Procédé selon l'une quelconque des revendications 1 à 7, dans lequel ledit matériau d'enzyme de restriction se fixe audit support à fonctionnalité azlactone à un pH compris entre 0 et 11 et à une température de 0 à 100°C, pendant un temps de réaction de 1 à 10 minutes.

9. Procédé selon l'une quelconque des revendications 1 à 8, dans lequel ladite séparation comprend une centrifugation, un écrémage, une filtration ou une sédimentation.
